# EUROPÄISCHE PATENTSCHRIFT

(11) **EP 3 013 394 B1**
(45) Veröffentlichungstag und Bekanntmachung des Hinweises auf die Patenterteilung: **26.03.2025**
(21) Anmeldenummer: 14734093.9
(22) Anmeldetag: 24.06.2014
(51) Int. Cl.: A61M 5/315, A61M 5/168, A61M 39/28, F16K 7/06

(54) **EINSTELLVORRICHTUNG FÜR EINEN DURCHFLUSSREGLER**
ADJUSTING DEVICE FOR A FLOW CONTROLLER
DISPOSITIF DE RÉGLAGE POUR RÉGULATEUR DE DÉBIT

(30) Priorität: 26.06.2013 DE 102013212325
(43) Veröffentlichungstag der Anmeldung: 04.05.2016
(73) Patentinhaber: B. Braun Melsungen AG, 34212 Melsungen (DE)
(72) Erfinder: HASLBECK, Karsten, 34212 Melsungen (DE)
(74) Vertreter: Patentanwälte Ruff, Wilhelm, Beier, Dauster & Partner mbB
(86) Internationale Anmeldenummer: PCT/EP2014/063312
(87) Internationale Veröffentlichungsnummer: WO 2014/206998

(56) Entgegenhaltungen:
- EP-A1- 0 295 075
- EP-A2- 0 730 876
- DE-U1- 7 811 534
- GB-A- 462 057
- US-A- 3 499 127
- US-A- 3 772 485
- US-A- 4 163 879

## Beschreibung

Die Erfindung betrifft eine Einstellvorrichtung zum Einstellen der Flussrate eines medizinischen Fluidfördersystems.

Ein medizinisches Fluidfördersystem kann beispielsweise ein Infusionsgerät zur Infusionstherapie oder zur Infusion eines Schmerzmittels in einen Patienten sein. Bei solchen Fluidfördersystemen ist es erforderlich, eine von mehreren möglichen Flussraten für die Fluidförderung auszuwählen und einzustellen. Die Einstellung der Flussrate, mit der das Fluid gefördert wird, muss mit hoher Genauigkeit und Sicherheit erfolgen. Eine Fehleinstellung kann vor allem bei Infusionsgeräten zu einer Über- oder Unterdosierung führen, die für einen Patienten kritische Auswirkungen haben kann.

Es ist bekannt, die Einstellung der Flussrate mit Hilfe eines Drehreglers vorzunehmen. Dabei dient ein Wählrad zum Wählen einer von mehreren möglichen Flussraten. Mit dem Wählrad ist eine Stellscheibe zum Einstellen der gewählten Flussrate rotatorisch fest verbunden. Mit dem radial äußeren Rand der Stellscheibe wirkt dabei eine Fangnase zusammen. Der Eingriff zwischen der Fangnase und der Stellscheibe ist dabei derart, dass die Fangnase die Stellscheibe und somit auch das Wählrad in derjenigen Drehstellung arretiert, die zur Einstellung der gewünschten Flussrate erforderlich ist.

Bei den bekannten Einstellvorrichtungen besteht eine Schwierigkeit darin, dass Zwischenstellungen zwischen benachbarten Drehstellungen entsprechender Flussraten eingestellt werden können, bei der weder die eine noch die andere Flussrate ausgewählt ist. Die versehentlich eingestellte Zwischenposition bleibt dabei eingestellt. Je nach Regulierungsmechanismus kann dies zu einer maximalen Förderung oder zu einer ausbleibenden Förderung des Fluids führen. Vor allem in der Infusionsmedizin kann dies kritische Folgen für einen Patienten haben.

Aus der EP 0 730 876 A ist eine Einstellvorrichtung mit den oberbegrifflichen Merkmalen des Anspruchs 1 bekannt. Bei der bekannten Einstellvorrichtung ist die Fangnase als flache dreieckige Spitze gestaltet, wobei der Spitzenwinkel der dreieckigen Spitze größer ist als der Winkel der Zickzacklinien der Stellscheibe.

Der Erfindung liegt die Aufgabe zugrunde, eine verbesserte Einstellvorrichtung zum Einstellen der Flussrate eines medizinischen Fluidfördersystems zu schaffen.

Die erfindungsgemäße Einstellvorrichtung ist definiert durch die Merkmale von Patentanspruch 1. Die Stellscheibe ist demnach sternförmig ausgebildet. Die Sternform führt zu einer rotationssymetrischen Form der Stellscheibe mit einem radial äußeren Rand, der mehrere Bereiche mit maximalem Radius und mehrere Bereiche mit minimalem Radius aufweist. Die Bereiche des radial äußeren Randes mit maximalem Radius können als Gipfel und die Bereiche mit minimalem Radius als Täler bezeichnet werden. Die Sternform der Stellscheibe führt dazu, dass ein Tal und ein Gipfel einander abwechseln. Das Zusammenwirken der Fangnase mit der Sternform der Stellscheibe führt zu stabilen Lagen der Stellscheibe, wenn die Fangnase mit einem Tal zusammengreift und zu instabilen Lagen, wenn die Fangnase mit einem Gipfel zusammengreift. Dabei ist entscheidend, dass ein Gipfel keinen konstanten Radius aufweist, sondern einen maximalen Radius, der zum benachbarten Tal hin abnimmt. Dadurch führt das Angreifen der Fangnase an einem Gipfel zu einer instabilen Drehstellung der Stellscheibe. Während die Täler den Drehstellungen zur Einstellung einer jeweiligen Flussrate dienen, entsprechen die dazwischen liegenden Gipfel den Drehstellungen zwischen benachbarten Drehpositionen einstellbarer Flussraten. Ein versehentliches Einstellen einer Drehposition zwischen benachbarten Flussraten führt somit zu einer instabilen Drehposition der Stellscheibe.

Eine Fangnase, die entgegen der Kraft einer Feder gelagert ist und von der Feder gegen den radial äußeren Rand der Stellscheibe gedrückt wird, wird bei Anliegen an dem radial äußeren Rand zwischen benachbarten Tälern dazu führen, dass die Federkraft die Stellscheibe aus ihrer instabilen Lage heraus in eine Drehung versetzt, um die Fangnase in ein Tal und somit in eine stabile Position zu bewegen. Erfindungsgemäß ist die Fangnase als Vorsprung an einer Biegefeder ausgebildet, nämlich als dreieckförmiger Vorsprung mit einer Spitze.

Die Sternform der Stellscheibe ergibt gemäß der Erfindung eine Zickzackform des radial äußeren Randes, wobei benachbarte Linien dieser Zickzackform in einem Winkel zwischen 10° und 155°, vorzugsweise zwischen 90° und 135°, zueinander geneigt angeordnet sind.

Im Folgenden wird anhand der Figuren ein Ausführungsbeispiel der Erfindung näher erläutert. Es zeigen:
- Fig. 1: eine Ansicht der Einstellvorrichtung aus Richtung der Rotationsachse,
- Fig. 2: eine Ansicht aus Richtung des Pfeils II in Fig. 1,
- Fig. 3: einen Schnitt entlang der Linie III-III in Fig. 2 in einer ersten Drehposition,
- Fig. 4: den Schnitt nach Fig. 3 in einer zweiten Drehposition und
- Fig. 5: das detailgemäß V in Fig. 4.

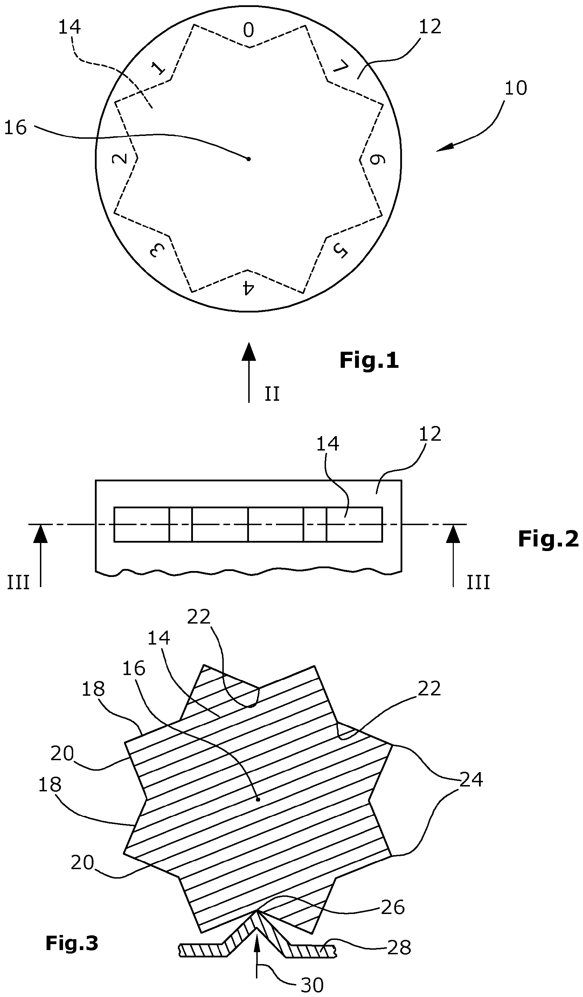

Fig. 1 zeigt eine Ansicht der Einstellvorrichtung 10, bei der die Oberfläche des Wählrades 12 aus Blickrichtung dessen Rotationsachse dargestellt ist. Entlang des radial äußeren Randes des Wählrades 12 sind die Ziffern 0-7 in gleichmäßigen Abständen zueinander dargestellt, um die diesen Ziffern entsprechenden Drehstellungen zu markieren. Jede der Ziffern 0-7 entspricht dabei einer möglichen einstellbaren Flussrate, wobei 0 keinem Fluss und 7 maximalem Fluss entspricht.

Das Wählrad 12 ist kreisrund in der Ansicht nach Fig. 1 ausgebildet und um eine Rotationsachse 16 rotierbar gelagert. Das Wählrad 12 ist mit einer rotationssymetrischen Stellscheibe 14 fest verbunden, sodass die Stellscheibe 14 um dieselbe Rotationsachse 16 rotieren kann. Das Wählrad 12 und die Stellscheibe 14 sind rotatorisch fest miteinander gekoppelt. In Fig. 1 ist die Stellscheibe 14 durch gestrichelte Linien als verdecktes Element hinter dem Wählrad 12 dargestellt.

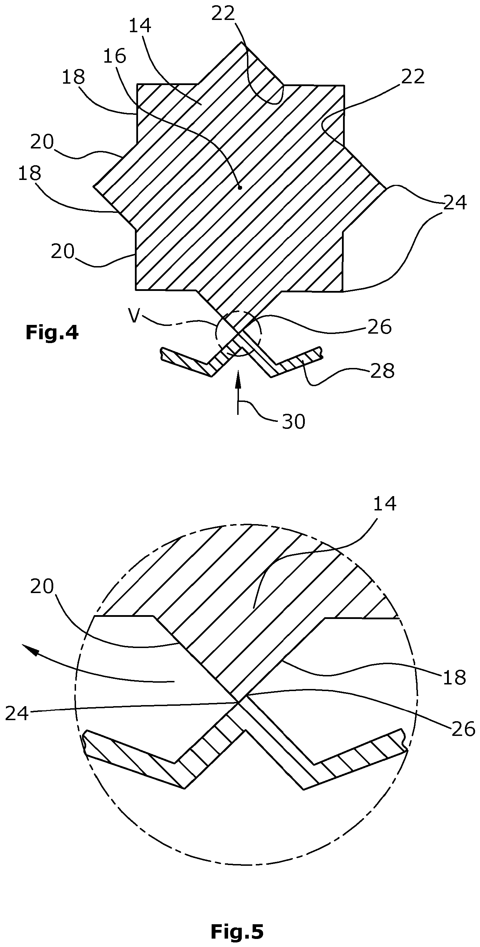

Die Stellscheibe 14 ist durch Zickzacklinien 18, 20 entlang ihres äußeren Umfanges sternförmig ausgebildet. Die Sternform ist aus Blickrichtung der Rotationsachse 16, wie in den Fign. 3 und 4 dargestellt, ersichtlich.

Die Zickzacklinien 18,20 des radial äußeren Randes der Stellscheibe 14 sind Geraden und zueinander in flachen Winkeln in einem Bereich zwischen ca. 110° und 130° angeordnet. Benachbarte Zickzacklinien 18, 20 bilden an ihren Berührungspunkten Täler 22 und Gipfel 24. Jeder Gipfel 24 bildet dabei als radial nach außen weisende Spitze einen Punkt mit maximalem Radius. In entsprechender Weise bildet jedes Tal 22 als radial nach innen weisende Spitze einen Punkt mit minimalem Radius. Die Radien aller Täler 22 sind jeweils gleich und die Radien aller Gipfel 24 sind jeweils gleich.

Mit den Zickzacklinien 18, 20 des sternförmigen radial äußeren Randes der Stellscheibe 14 greift eine Fangnase 26 zusammen, die wie in den Fign. 3 - 5 dargestellt ist, als dreieckige Spitze einer Biegefeder 28 ausgebildet ist. In dem Ausführungsbeispiel handelt es sich bei der Biegefeder 28 um eine geknickte Blattfeder. Dabei ist die Biegefeder 28 in Bezug auf die Stellscheibe 14 derart angeordnet, dass die Federkraft die Fangnase 26 in Richtung des Pfeiles 30 in den Fign. 3 und 4 radial nach innen gegen die Stellscheibe 14 drückt.

Fig. 3 zeigt die stabile Lage der Stellscheibe 14, in der die Feder 28 die Fangnase 26 in ein Tal 22 der Sternform presst. Bei Rotation der Stellscheibe 14 durch Verdrehen des Wählrades 12 ist eine Kraft aufzubringen, die der derjenigen Kraft entgegen wirkt, die die Biegefeder 28 über die Fangnase 26 auf den äußeren Rand der Stellscheibe 14 ausübt. Bei versehentlichem Einstellten einer Zwischenposition zwischen benachbarten Tälern befindet sich die Stellscheibe 14 immer in einer instabilen Lage. Eine solche Zwischenposition ist in den Fign. 4 und 5 dargestellt. Dabei drückt die Fangnase 26 mit der Kraft der Biegefeder 28 aufgrund der Sternform schräg gegen den Rand der Stellscheibe 14, wodurch die Stellscheibe 14 in Rotation versetzt wird, bis die Fangnase in einem der Täler 22 zu liegen kommt. Das Einstellen einer Zwischenposition zwischen benachbarten Tälern ist dadurch verhindert.

## Patentansprüche

1. Einstellvorrichtung (10) zum Einstellen der Flussrate eines medizinischen Fluidfördersystems, mit einem Wählrad (12) zum Wählen einer von mehreren möglichen Flussraten, einer mit dem Wählrad (12) verbundenen Stellscheibe (14) zum Einstellen der gewählten Flussrate und einer mit dem äußeren Rand der Stellscheibe (14) zusammen wirkenden Fangnase (26), wobei die Stellscheibe (14) durch Zickzacklinien (18, 20) entlang ihres äußeren Randes sternförmig ausgebildet ist, wobei die Zickzacklinien (18, 20) Geraden und zueinander in einem Winkel zwischen 10° und 155° angeordnet sind, wobei benachbarte Zickzacklinien (18, 20) einander abwechselnde Gipfel (24) und Täler (22) bilden, wobei die Stellscheibe (14) rotatorisch fest mit dem Wählrad (12) gekoppelt ist, wobei die Fangnase (26) derart entgegen der Kraft einer Biegefeder (28) gelagert ist, dass ein Eingriff der Fangnase (26) in ein Tal (22) eine stabile und ein Eingriff der Fangnase (26) mit dem Gipfel (24) eine instabile Lage der Stellscheibe (14) bewirkt, und wobei die Fangnase (26) als dreieckige Spitze der Biegefeder (28) ausgebildet ist, **dadurch gekennzeichnet, dass** ein Spitzenwinkel der dreieckigen Spitze kleiner ist als der Winkel der Zickzacklinien (18, 20).

2. Einstellvorrichtung (10) nach Anspruch 1, **dadurch gekennzeichnet, dass** jedem Tal (22) die zur Einstellung einer Flussrate erforderliche Drehstellung des Wählrades (12) und der Stellscheibe (14) entspricht.

3. Einstellvorrichtung (10) nach Anspruch 1 oder 2, **dadurch gekennzeichnet, dass** jedem Gipfel (24) die Drehstellung des Wählrades (12) und der Stellscheibe (14) genau zwischen zwei benachbarten Drehstellungen entsprechender Flussraten entspricht.

4. Einstellvorrichtung (10) nach einem der vorhergehenden Ansprüche, **dadurch gekennzeichnet, dass** die Gipfel (24) und/oder die Täler (22) jeweils abgerundet sind.

## Claims

1. Adjusting device (10) for adjusting the flow rate of a medical fluid delivery system, comprising a selector wheel (12) for selecting one of a plurality of possible flow rates, a setting disc (14), which is connected to the selector wheel (12), for adjusting the selected flow rate, and a catch nose (26) which interacts with the outer periphery of the setting disc (14), wherein the setting disc (14) is formed so as to be star-shaped by zigzag lines (18, 20) along its outer periphery, wherein the zigzag lines (18, 20) are straight lines and disposed relative to one another at an angle between 10° and 155°, wherein adjacent zigzag lines (18, 20) form alternating peaks (24) and troughs (22), wherein the setting disc (14) is co-rotationally coupled to the selector wheel (12), wherein the catch nose (26) is mounted counter to the force of a bending spring (28) in such a manner that an engagement of the catch nose (26) in a trough (22) causes a stable, and an engagement of the catch nose (26) with the peak (24) causes an unstable, position of the setting disc (14), and wherein the catch nose (26) is formed as a triangular tip of the bending spring (28), **characterized in that** a tip angle of the triangular tip is smaller than the angle of the zigzag lines (18, 20).

2. Adjusting device (10) according to Claim 1, **characterized in that** each trough (22) corresponds to the rotational position of the selector wheel (12) and of the setting disc (14) required for setting a flow rate.

3. Adjusting device (10) according to Claim 1 or 2, **characterized in that** each peak (24) corresponds to the rotational position of the selector wheel (12) and of the setting disc (14) exactly between two adjacent rotational positions of corresponding flow rates.

4. Adjusting device (10) according to one of the preceding claims, **characterized in that** the peaks (24) and/or the troughs (22) are each radiused.

## Revendications

1. Dispositif de réglage (10) permettant de régler le débit d'un système de transport de fluide médical, comprenant une molette de sélection (12) pour sélectionner l'un de plusieurs débits possibles, un disque de réglage (14) relié à la molette de sélection (12) pour régler le débit sélectionné, et un nez d'arrêt (26) coopérant avec le bord extérieur du disque de réglage (14), dans lequel le disque de réglage (14) est réalisé en forme d'étoile par des lignes en zigzag (18, 20) le long de son bord extérieur, les lignes en zigzag (18, 20) étant des droites et étant disposées selon un angle compris entre 10° et 155° l'une par rapport à l'autre, dans lequel des lignes en zigzag voisines (18, 20) forment des sommets (24) et des creux (22) en alternance, le disque de réglage (14) étant couplé à la molette de sélection (12) de manière verrouillée en rotation, dans lequel le nez d'arrêt (26) est monté contre la force d'un ressort de flexion (28) de telle sorte qu'une mise en prise du nez d'arrêt (26) avec un creux (22) provoque une position stable et une mise en prise du nez d'arrêt (26) avec un sommet (24) provoque une position instable du disque de réglage (14), et dans lequel le nez d'arrêt (26) est réalisé comme une pointe triangulaire du ressort de flexion (28), **caractérisé en ce qu'**un angle de pointe de la pointe triangulaire est inférieur à l'angle des lignes en zigzag (18, 20).

2. Dispositif de réglage (10) selon la revendication 1, **caractérisé en ce qu'**à chaque creux (22) correspond la position de rotation de la molette de sélection (12) et du disque de réglage (14) nécessaire au réglage d'un débit.

3. Dispositif de réglage (10) selon la revendication 1 ou 2, **caractérisé en ce qu'**à chaque sommet (24) correspond la position de rotation de la molette de sélection (12) et du disque de réglage (14) exactement entre deux positions de rotation voisines des débits correspondants.

4. Dispositif de réglage (10) selon l'une quelconque des revendications précédentes, **caractérisé en ce que** les sommets (24) et/ou les creux (22) sont respectivement arrondis.
